(19) 

**Europäisches Patentamt**

**European Patent Office**

**Office européen des brevets**

(11) **EP 4 613 837 A1**

(12) **EUROPEAN PATENT APPLICATION**
published in accordance with Art. 153(4) EPC

(43) Date of publication:
**10.09.2025 Bulletin 2025/37**

(21) Application number: **23885892.2**

(22) Date of filing: **06.11.2023**

(51) International Patent Classification (IPC):
*C12M 1/00* (2006.01)    *C12M 1/34* (2006.01)
*C12M 3/00* (2006.01)    *C12N 5/076* (2010.01)

(52) Cooperative Patent Classification (CPC):
**C12M 1/00; C12M 1/34; C12M 3/00; C12N 5/06**

(86) International application number:
**PCT/JP2023/039902**

(87) International publication number:
**WO 2024/096138 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **04.11.2022 JP 2022177449**

(71) Applicants:
• **Mitsui Chemicals, Inc.**
**Tokyo 104-0028 (JP)**
• **Tokai University Educational System**
**Tokyo, 151-0063 (JP)**
• **OSAKA UNIVERSITY**
**Suita-shi**
**Osaka 565-0871 (JP)**
• **Public University Corporation Yokohama City
University**
**Yokohama-shi, Kanagawa 236-0027 (JP)**

(72) Inventors:
• **KIMURA Hiroshi**
**Hiratsuka-shi, Kanagawa 259-1292 (JP)**

• **SHIRAI Hiroki**
**Hiratsuka-shi, Kanagawa 259-1292 (JP)**
• **NAKAMURA Hiroko**
**Hiratsuka-shi, Kanagawa 259-1292 (JP)**
• **IKAWA Masahito**
**Suita-shi, Osaka 565-0871 (JP)**
• **FUJIWARA KAMOSHITA Maki**
**Suita-shi, Osaka 565-0871 (JP)**
• **OGAWA Takehiko**
**Yokohama-shi, Kanagawa 236-0004 (JP)**
• **YAMAUCHI ISHIKAWA Yu**
**Yokohama-shi, Kanagawa 236-0004 (JP)**
• **NAGATA Shino**
**Yokohama-shi, Kanagawa 236-0004 (JP)**
• **ESASHIKA Katsuhiro**
**Tokyo 104-0028 (JP)**
• **YANG Jingjing**
**Tokyo 104-0028 (JP)**
• **YAMASAKI Satoshi**
**Tokyo 104-0028 (JP)**

(74) Representative: **J A Kemp LLP**
**80 Turnmill Street**
**London EC1M 5QU (GB)**

(54) **CULTURE DEVICE, METHOD FOR MONITORING CULTURE SUBJECT, AND CULTURE CHIP**

(57)    The culture device includes a container main body that accommodates a culture solution and a culture chip that forms a culture room inside the container main body, in which the container main body has a bottom wall portion having light transmittance and gas permeability, and the culture chip has a frame portion that abuts on an upper surface of the bottom wall portion, and a membrane member that has liquid permeability or liquid-containing substance permeability, is fixed to an upper surface of the frame portion, and forms the culture room with the bottom wall portion and the frame portion.

EP 4 613 837 A1

**(Cont. next page)**

FIG. 1

**Description**

TECHNICAL FIELD

The present invention relates to a culture device, a method for monitoring a culture subject, and a culture chip.

Priority is claimed on Japanese Patent Application No. 2022-177449, filed November 4, 2022, the content of which is incorporated herein by reference.

BACKGROUND ART

Patent Document 1 discloses a culture medium additive containing agarose having a weight-average molecular weight of 10,000 to 60,000, and a method for culturing cells or tissues, in which cells or tissues are cultured in a state of being dispersed in the culture medium composition.

Citation List

Patent Document

Patent Document 1: Japanese Unexamined Patent Application, First Publication No. 2022-25134

SUMMARY OF INVENTION

Technical Problem

In recent years, severe declining birthrate has progressed, the number of patients with infertility has increased, and about half of patients with infertility have male infertility. Although about 90% of the male infertility is due to a spermatogenesis disorder, due to the complexity of the spermatogenesis phenomenon, there are many unknown parts in the details of the spermatogenesis mechanism. The fact that there is almost no effective treatment method for male infertility at present is also one of the supporting evidences.

Spermatozoa are formed through cell division, differentiation, and meiosis of spermatogonia in a spatiotemporal manner in seminiferous tubules having a diameter of less than 200 μm in the testis. In the field of infertility treatment or the field of embryology, the aim is to elucidate the spermatogenesis mechanism by monitoring the spatiotemporal differentiated morphological changes from the spermatogonia to the spermatozoa.

As a method of monitoring the entire process of spermatogenesis, an in vitro (ex vivo) experimental system as described in Patent Document 1 has been proposed. As this in vitro experimental system, for example, there is a method of placing testicular tissue excised from a mouse on an agarose gel block (agar) immersed in a culture medium and culturing the tissue (a conventional organ culture method). According to this method, long-term culture for a few months can be performed, and spermatogenesis ex vivo can be realized.

In a case of monitoring a tissue in the organ culture method in the related art, the state of the tissue is simply monitored using an stereoscopic microscope. On the other hand, to monitor the process of forming spermatozoa having a size of less than 10 μm in detail, it is necessary to perform bright field and fluorescence monitoring using a high-magnification lens of an inverted microscope.

However, in the above-described organ culture method of the related art, the thickness and color of the agarose gel block hinder optical monitoring, and thus it is difficult to perform high-definition monitoring with an inverted microscope while culturing. Therefore, at present, in a case where detailed monitoring is required, it is necessary to move the testicular tissue to a plate for monitoring and perform bright field and fluorescence monitoring with an inverted microscope.

However, this operation has a serious disadvantage of not obtaining time resolution of monitoring in addition to damaging the testicular tissue or causing contamination.

An object of the present invention is to provide a culture device, a method for monitoring a culture subject, and a culture chip, which solve the above-described issues. Solution to Problem

A culture device according to an aspect of the present invention includes a container main body that accommodates a culture solution and a culture chip that forms a culture room inside the container main body, in which the container main body has a bottom wall portion having light transmittance and gas permeability, and the culture chip has a frame portion that abuts on an upper surface of the bottom wall portion, and a membrane member that has liquid permeability or liquid-containing substance permeability, is fixed to an upper surface of the frame portion, and forms the culture room with the bottom wall portion and the frame portion.

In the culture device, the oxygen in a gas phase may be supplied to a culture subject housed in the culture room through the bottom wall portion, and the culture solution may be supplied to the culture subject through the membrane member.

In the culture device, the thickness of the culture subject housed in the culture room may be controlled by the height of the frame portion.

In the culture device, the membrane member may have light transmittance.

In the culture device, the frame portion may be fixed to the upper surface of the bottom wall portion. In the culture device, the frame portion may have a height within a range of 20 μm or more and 400 μm or less.

In the culture device, the culture subject is a seminiferous tubule, and the frame portion may have a height within a range of 50 μm or more and 350 μm or less.

In the culture device, the culture subject is a testicular tissue of a mouse, and the frame portion may have a height within a range of 50 μm or more and 200 μm or less.

In the culture device, the upper surface of the bottom wall portion may have hydrophobicity.

In the culture device, a part of the upper surface of the bottom wall portion, that forms the culture room, may have hydrophobicity, and a part other than the part that forms the culture room may have hydrophilicity.

A method for monitoring a culture subject according to an aspect of the present invention includes monitoring the culture room through the bottom wall portion using the culture device set on an inverted microscope.

A culture chip according to an aspect of the present invention includes a membrane member having light transmittance or liquid permeability and a frame portion fixed to one surface of the membrane member.

In the culture chip, the membrane member may have light transmittance.

A cell culture method according to an aspect of the present invention is a cell culture method using the culture device, in which the oxygen in a gas phase is supplied to a culture subject housed in the culture room through the bottom wall portion, and the culture solution is supplied to the culture subject through the membrane member. Advantageous Effects of Invention

According to the aspect of the present invention, it is possible to obtain the time resolution of monitoring without damaging the culture subject or causing contamination.

BRIEF DESCRIPTION OF DRAWINGS

[FIG. 1] A configuration diagram of a culture device according to a first embodiment.
[FIG. 2] A bottom view of a culture chip according to the first embodiment.
[FIG. 3] An explanatory diagram of a method for monitoring a culture subject according to the first embodiment.
[FIG. 4] A configuration diagram of a culture device according to a comparative example.
[FIG. 5] A diagram showing results of a testicular tissue culture experiment using a culture device according to the comparative example.
[FIG. 6] A diagram showing results of a testicular tissue culture experiment using a culture device according to a first example.
[FIG. 7] A graph showing postnatal age and seminiferous tubule growth rate of testicular tissues according to the first example and the comparative example.
[FIG. 8] A table summarizing a relationship between a pore diameter and visibility of a culture subject in a case where polycarbonate is used as the membrane member.

[FIG. 9] A comparison photograph of a bright field and a fluorescence field (RFP) using an inverted microscope in a case where polycarbonate having a pore diameter of 0.4 μm is used as a membrane member.
[FIG. 10] A comparison photograph of a bright field and a fluorescence field (RFP) using an inverted microscope in a case where polycarbonate having a pore diameter of 10 μm is used as a membrane member.
[FIG. 11] A table summarizing a relationship between a pore diameter and visibility of a culture subject in a case where polyethylene terephthalate is used as the membrane member.
[FIG. 12] A comparison photograph of a bright field and a fluorescence field (RFP) using an inverted microscope in a case where polyethylene terephthalate having a pore diameter of 0.45 μm is used as a membrane member.
[FIG. 13] A comparison photograph of a bright field and a fluorescence field (RFP) using an inverted microscope 30 in a case where polyethylene terephthalate having a pore diameter of 3 μm is used as a membrane member.
[FIG. 14] A table summarizing the materials, pore diameters, porosities, and light transmittances of the membrane members used in the first example.
[FIG. 15] A graph showing postnatal age and Acr-GFP expression rate of testicular tissues according to a second example and the comparative example.
[FIG. 16] A diagram comparing fluorescence images of testicular tissues at the postnatal age (35th day) in a case where polydimethylsiloxane (PDMS) is used as a bottom wall portion, with Comparative Example.
[FIG. 17] A diagram comparing fluorescence images of testicular tissues at postnatal age (35th day) in a case where TPX (registered trademark) which is a 4-methyl-1-pentene-based polymer and LumoX (registered trademark) which is a tetrafluoroethylene-hexafluoropropylene copolymer (FEP) are used as a bottom wall portion.
[FIG. 18] A diagram showing a state in which a culture solution and a culture subject are set in a culture device according to a third embodiment.
[FIG. 19] A diagram showing a state in which a culture solution and a culture subject are set in a culture device in which an upper surface of a bottom wall portion is hydrophilic.
[FIG. 20] A diagram showing a state in which a culture solution and a culture subject are set in a culture device according to a modification example of the third embodiment.
[FIG. 21] A plan view of the container main body shown in FIG. 20.

DESCRIPTION OF EMBODIMENTS

Hereinafter, embodiments of the present invention will be described below with reference to the drawings.

(First embodiment)

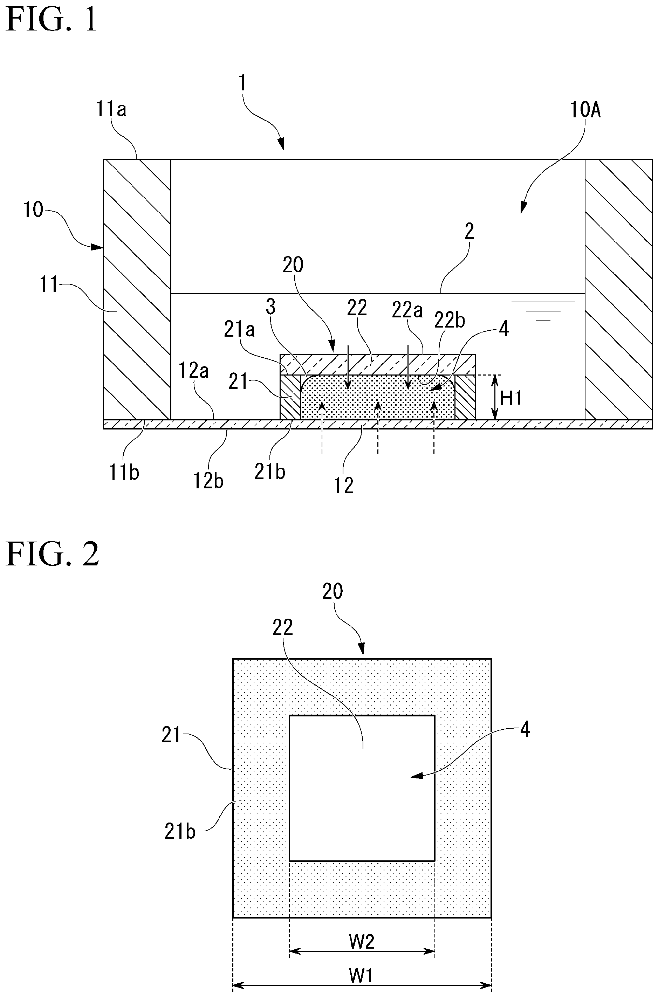

FIG. 1 is a configuration diagram of a culture device 1 according to a first embodiment. As shown in FIG. 1, the culture device 1 includes a container main body 10 and a culture chip 20.

The container main body 10 forms a culture solution accommodation chamber 10A that accommodates the culture solution 2 therein. The culture chip 20 is disposed in the culture solution accommodation chamber 10A and forms a culture room 4 for culturing the culture subject 3. In the present embodiment, a testicular tissue is exemplified as the culture subject 3. It is noted that the culture subject 3 may be, for example, other organ tissues such as an ovary tissue, a liver tissue, a kidney tissue, a pancreas tissue, a lung tissue, and an intestinal tract tissue, or may be a tissue piece (a piece of tissue diced), a cell mass (an organoid, a spheroid, or the like), a cell, a bacterium, or the like.

The container main body 10 is formed in a bottomed cylindrical shape. It is noted that the container main body 10 shown in FIG. 1 is a single container, but may have a configuration in which a plurality of container main bodies 10 are connected, such as a multi-well plate. The container main body 10 has a side wall portion 11 and a bottom wall portion 12. The side wall portion 11 is formed in a tubular shape surrounding the periphery of the culture solution accommodation chamber 10A. The side wall portion 11 may have a cylindrical shape or may have a rectangular tubular shape. An opening on the upper surface 11a side of the side wall portion 11 is open, and an opening on the lower surface 11b side of the side wall portion 11 is closed by the bottom wall portion 12.

The bottom wall portion 12 has light transmittance and gas permeability. The material of the bottom wall portion 12 is polydimethylsiloxane (PDMS), which is a type of silicone. It is noted that the material of the bottom wall portion 12 is not limited to polydimethylsiloxane as long as the bottom wall portion 12 has light transmittance and gas permeability. For example, a 4-methyl-1-pentene-based polymer or a tetrafluoroethylene-hexafluoropropylene copolymer (FEP) can also be used as the material of the bottom wall portion 12. The light transmittance of the bottom wall portion 12 is preferably colorless and transparent, and the gas permeability of the bottom wall portion 12 is only required to allow at least oxygen and carbon dioxide to be permeated. Specifically, the oxygen permeability of the bottom wall portion 12 at a temperature of 23°C and a humidity of 0% is 4,500 to 90,000 $cm^3/(m^2 \times 24\ h \times atm)$, preferably 4,500 to 67,500 $cm^3/(m^2 \times 24\ h \times atm)$, more preferably 4,500 to 47,000 $cm^3/(m^2 \times 24\ h \times atm)$, and still more preferably 4,500 to 45,000 $cm^3/(m^2 \times 24\ h \times atm)$. The upper surface 12a of the bottom wall portion 12 is fixed to the lower surface 11b of the side wall portion 11 by adhesion or the like.

The material of the side wall portion 11 is not particularly limited as long as the material can maintain the shape of the culture solution accommodation chamber 10A. Examples of a material of the side wall portion 11 include polycarbonate (PC), polyester (PE), polystyrene (PS), glass, and the like. The side wall portion 11 is preferably colorless and transparent, but may be colored and may not have light transmittance. In a case where the side wall portion 11 is colored, black is preferable to prevent autofluorescence or reflection.

The culture chip 20 is formed in a topped cylindrical shape. The culture chip 20 has a frame portion 21 and a membrane member 22. The frame portion 21 abuts on the upper surface 12a of the bottom wall portion 12 of the container main body 10. The lower surface 21b of the frame portion 21 abuts on the upper surface 12a of the bottom wall portion 12 by adhesion. It is noted that the frame portion 21 may be in a state of being placed on the upper surface 12a of the bottom wall portion 12 as long as the frame portion 21 abuts on the upper surface 12a of the bottom wall portion 12, may be in a state of being closely attached to the upper surface 12a of the bottom wall portion 12 by Van der Waals force, or may be in a state of being bonded with an adhesive or the like.

FIG. 2 is a bottom view of a culture chip 20 according to the first embodiment. The culture chip 20 shown in FIG. 2 has a frame portion 21 having a rectangular frame shape in bottom view. The shape of the frame portion 21 is not limited to a rectangular frame shape, and the culture room 4 is only required to be formed inside the frame portion 21. Examples other than the rectangular frame shape include a polygonal frame shape and a circular frame shape. It is noted that in the present embodiment, the frame portion 21 is formed in a frame shape of a square in bottom view.

The size of the outer shape of the frame portion 21 is only required to be a size such that the frame portion 21 can be disposed in the culture solution accommodation chamber 10A of the container main body 10. For example, the width W1 of one side of the square outer edge of the frame portion 21 is within a range of 10 mm or more and 20 mm or less (for example, 15 mm). In addition, for example, the width W2 of one side of the square inner edge of the frame portion 21 is within a range of 2 mm or more and less than 10 mm (for example, 7 mm).

Returning to FIG. 1, the frame portion 21 has a height H1 for controlling the thickness of the cultured tissue to be cultured in the culture room 4. The height H1 of the frame portion 21 is a dimension from the upper surface 21a to the lower surface 21b, and is preferably within a range of 50 $\mu$m or more and 200 $\mu$m or less in a case where the culture subject 3 is a testicular tissue of a mouse. In Examples described later, the testicular tissue of a mouse is used as the culture subject 3, but the aperture of the seminiferous tubule of the adult of various animals is about 350 $\mu$m in a rat, about 200 $\mu$m in a mouse, about 300 $\mu$m in a monkey, and 220 $\mu$m to 250

μm in a human. Therefore, in a case where the culture subject 3 is a seminiferous tubule regardless of various animals, it is preferable to set the height H1 of the frame portion 21 within a range of 50 μm or more and 350 μm or less to control the thickness of the seminiferous tubule. In a case of a tissue or the like other than the mouse testicular tissue, it is difficult to reduce the height of the culture subject 3 to less than 20 μm, and in a case of a large tissue or the like, it has been confirmed that nutrients enter the tissue or the like by diffusion up to a height of 400 μm. Therefore, it is preferable to set the height H1 of the frame portion 21 within a range of 20 μm or more and 400 μm or less in accordance with the type of the culture subject 3 and to control the thickness of the culture subject 3.

In a case where the culture subject 3 is enlarged, oxygen (indicated by a dotted arrow in FIG. 1) and the culture solution 2 (indicated by a solid arrow in FIG. 1) do not reach the central portion thereof, and the central portion may be necrotic. On the other hand, by controlling the thickness of the culture subject 3 by the height H1 of the frame portion 21, oxygen and the culture solution 2 can be made to reach the center portion of the culture subject 3, and necrosis of the center portion can be avoided. The height H1 of the frame portion 21 may be set according to the type of the culture subject 3.

It is more preferable that the membrane member 22 has liquid permeability or liquid-containing substance permeability and further has light transmittance. The material of the membrane member 22 is polycarbonate (PC) or polyethylene terephthalate (PET). The material of the membrane member 22 is not limited to polycarbonate or polyethylene terephthalate as long as the membrane member 22 has liquid permeability or liquid-containing substance permeability. For example, polytetrafluoroethylene (PTFE), nylon, nitrocellulose, polyvinylidene fluoride (PVDF), and polyimide (PI) may be used, and the form of the membrane member 22 may be a mesh membrane such as a porous membrane or a nonwoven fabric, or a dialysis membrane.

In addition, the material of the membrane member 22 may be any material as long as it has no toxicity to the culture subject 3 and can control the thickness of the culture subject 3. The light transmittance of the membrane member 22 is preferably colorless and transparent, and the membrane member 22 may have liquid permeability capable of allowing at least the culture solution 2 to be permeated, or may have liquid-containing substance permeability capable of allowing a liquid substance in the culture solution 2, for example, a liquid-containing substance such as glucose or a liquid factor, to be permeated. The lower surface 22b of the membrane member 22 is fixed to the upper surface 21a of the frame portion 21, and forms the culture room 4 together with the bottom wall portion 12 and the frame portion 21.

The material of the frame portion 21 is not particularly limited as long as it has no toxicity to the culture subject 3 and can maintain the shape of the culture solution accommodation chamber 10A. As the material of the frame portion 21, polydimethylsiloxane (PDMS) having light transmittance may be used, as in the bottom wall portion 12. In this case, the frame portion 21 and the membrane member 22 may be covalently bonded to each other by a silane coupling treatment.

In addition, the frame portion 21 may not have light transmittance and may be a tape material having a pressure-sensitive adhesive surface on the upper surface 21a and the lower surface 21b. That is, the frame portion 21 and the membrane member 22 may be fixed by a pressure sensitive adhesive. As the tape material forming the frame portion 21, a polyimide tape in which a base material is polyimide (PI) and a silicone-based pressure sensitive adhesive is provided on an upper surface 21a and a lower surface 21b is preferable.

According to the culture device 1 having the above-described configuration, the culture subject 3 can be cultured by preparing the container main body 10, sequentially placing the culture subject 3 and the culture chip 20 on the bottom wall portion 12 of the container main body 10, and introducing the culture solution 2 into the container main body 10. Oxygen (indicated by a dotted arrow in FIG. 1) in a gas phase is supplied to the culture subject 3 accommodated in the culture room 4 via the bottom wall portion 12, and the culture solution 2 (indicated by a solid arrow in FIG. 1) is supplied via the membrane member 22. As a result, it is possible to monitor the spatiotemporal morphological change of the culture subject 3 while controlling the thickness of the culture subject 3.

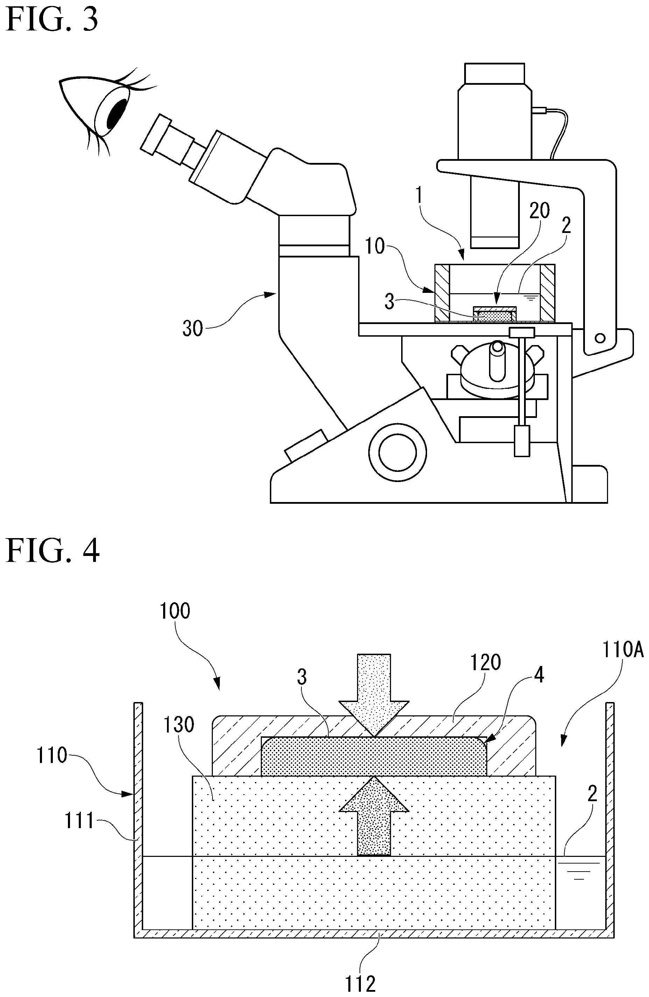

FIG. 3 is an explanatory diagram of a method for monitoring a culture subject according to the first embodiment. In the method for monitoring a culture subject according to the present embodiment, for example, as shown in FIG. 3, the culture device 1 is set on an inverted microscope 30, and the culture room 4 is monitored.

As shown in FIG. 1, the bottom wall portion 12 of the container main body 10 has light transmittance and gas permeability for culturing and monitoring the culture subject 3. The culture chip 20 is obtained by adhering a membrane member 22 to an upper surface 21a of a thin sheet-shaped frame portion 21 having no toxicity. Since the membrane member 22 has light transmittance in the same manner as the bottom wall portion 12, the membrane member 22 do not hinder to monitoring the inside of the culture room 4 through the bottom wall portion 12 by the inverted microscope 30.

Since the culture device 1 does not use an agarose gel (agar) used in the organ culture method in the related art, the experimental preparation and operability are simpler than those in the related art. Furthermore, since the culture chip 20 is easy to be produced and has a wide range of options such as a culture range, the thickness of a sheet, and a type of a membrane member 22, the culture chip 20 can be applied to culture of not only a testicular tissue but also other organ tissues or tissue pieces (pieces obtained by dicing a tissue), a cell mass (organoids, spheroids, and the like), cells, bacteria, and the like.

As described above, according to the culture device 1 according to the present embodiment, it is possible to perform high-accuracy optical monitoring using a high-magnification lens of the inverted microscope 30, and the culture device 1 can be set as it is on the inverted microscope 30, thereby the operability is improved as compared with the related art. As a result, for example, the spatiotemporal morphological change of the cell in the spermatogenesis can be captured in detail by live imaging or the like. Therefore, for example, it is possible to contribute to drug discovery, medicine, life science research, and the like for the purpose of elucidating a treatment method for spermatogenesis disorder by analyzing the essence of spermatogenesis using machine learning (AI).

As described above, the culture device 1 according to the present embodiment includes a container main body 10 that accommodates a culture solution 2 and a culture chip 20 that forms a culture room 4 inside the container main body 10, in which the container main body 10 has a bottom wall portion 12 having light transmittance and gas permeability, and the culture chip 20 has a frame portion 21 that abuts on an upper surface 12a of the bottom wall portion 12, and a membrane member 22 that has liquid permeability or liquid-containing substance permeability, is fixed to an upper surface 21a of the frame portion 21, and forms the culture room 4 with the bottom wall portion 12 and the frame portion 21. According to this configuration, it is possible to obtain the time resolution of monitoring without damaging the culture subject 3 or causing contamination.

In addition, in the present embodiment, the thickness of the culture subject 3 accommodated in the culture room 4 is controlled by the height of the frame portion 21. According to this configuration, necrosis in the central portion of the culture subject 3 can be suppressed.

In addition, in the present embodiment, the frame portion 21 is fixed to the upper surface 12a of the bottom wall portion 12. According to this configuration, the misregistration of the culture chip 20 in the culture solution accommodation chamber 10A or the floating of the culture chip 20 can be suppressed.

In addition, in the present embodiment, the membrane member 22 has light transmittance. According to this configuration, the culture subject 3 can be easily monitored in the bright field.

In addition, in the present embodiment, the material of the bottom wall portion 12 is polydimethylsiloxane. According to this configuration, high light transmittance and high gas permeability can be imparted to the bottom wall portion 12.

In addition, in the present embodiment, the material of the membrane member 22 is polycarbonate or polyethylene terephthalate. According to this configuration, high light transmittance and high liquid permeability can be imparted to the membrane member 22.

In addition, in the present embodiment, oxygen is supplied through the bottom wall portion 12, and the culture solution 2 is supplied through the membrane member 22 to the culture subject 3 accommodated in the culture room 4. According to this configuration, it is possible to monitor the spatiotemporal morphological change of the culture subject 3 while controlling the thickness of the culture subject 3.

In addition, in the present embodiment, the culture subject 3 is a testicular tissue. According to this configuration, it is possible to capture the spatiotemporal morphological change of the cell in the spermatogenesis in detail by live imaging or the like.

In addition, in the present embodiment, the frame portion 21 has a height H1 within a range of 50 $\mu$m or more and 200 $\mu$m or less. According to this configuration, it is possible to suppress necrosis of the central portion of the culture subject 3 during the culture.

A method for monitoring a culture subject according to the present embodiment includes monitoring the culture room 4 through the bottom wall portion 12 using the culture device 1 set on an inverted microscope 30. According to this configuration, it is possible to perform high-accuracy optical monitoring using a high-magnification lens of the inverted microscope 30, and the culture device 1 can be set as it is on the inverted microscope 30, thereby the operability is improved as compared with the related art.

In addition, the culture chip 20 according to the present embodiment has the membrane member 22 having liquid permeability or liquid-containing substance permeability, and the frame portion 21 fixed to one surface (the lower surface 22b) of the membrane member 22. According to this configuration, since the culture chip 20 is easy to be produced and has a wide range of options such as a culture range, the thickness of a sheet, and a type of a membrane member 22, the culture chip 20 can be applied to culture of not only a testicular tissue but also other organ tissues or tissue pieces (pieces obtained by dicing a tissue), a cell mass (organoids, spheroids, and the like), cells, bacteria, and the like.

[First example]

Hereinafter, a first example of the present invention will be described. The present invention is not limited to the following first example, and can be appropriately modified and implemented within the scope not departing from the gist thereof. In the following description, the same or equivalent configurations as those in the above-described embodiment are denoted by the same reference numerals, and descriptions thereof will be simplified or omitted.

First, a comparative example will be described for comparison with the first example of the present invention.

FIG. 4 is a configuration diagram of a culture device 100 according to a comparative example. As shown in FIG. 4, a culture device 100 according to the comparative example includes a container main body 110, a culture chip 120, and an agarose gel 130.

The container main body 110 forms a culture solution accommodation chamber 110A that accommodates the culture solution 2 therein. The container main body 110 is formed in a bottomed cylindrical shape and has a side wall portion 111 and a bottom wall portion 112. An agarose gel 130 is placed on the bottom wall portion 112. The upper surface of the agarose gel 130 is positioned above the liquid level of the culture solution 2 and supports the culture subject 3. The reason why the culture subject 3 is positioned above the liquid surface is that the culture subject 3 is necrotic in a case where oxygen is not supplied.

A culture chip 120 that forms a culture room 4 for culturing a culture subject 3 is placed on an upper surface of the agarose gel 130. The culture chip 120 has light transmittance and gas permeability. As a result, oxygen can be supplied to the culture subject 3 through the culture chip 120, and the culture solution 2 that is sucked through the agarose gel 130 can be supplied to the culture subject 3.

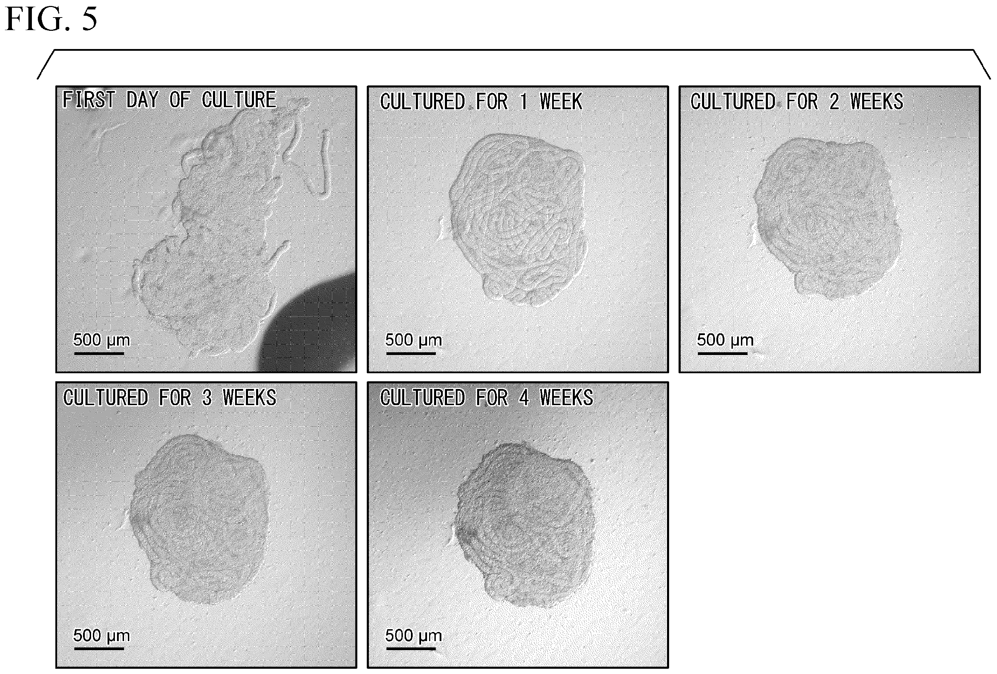

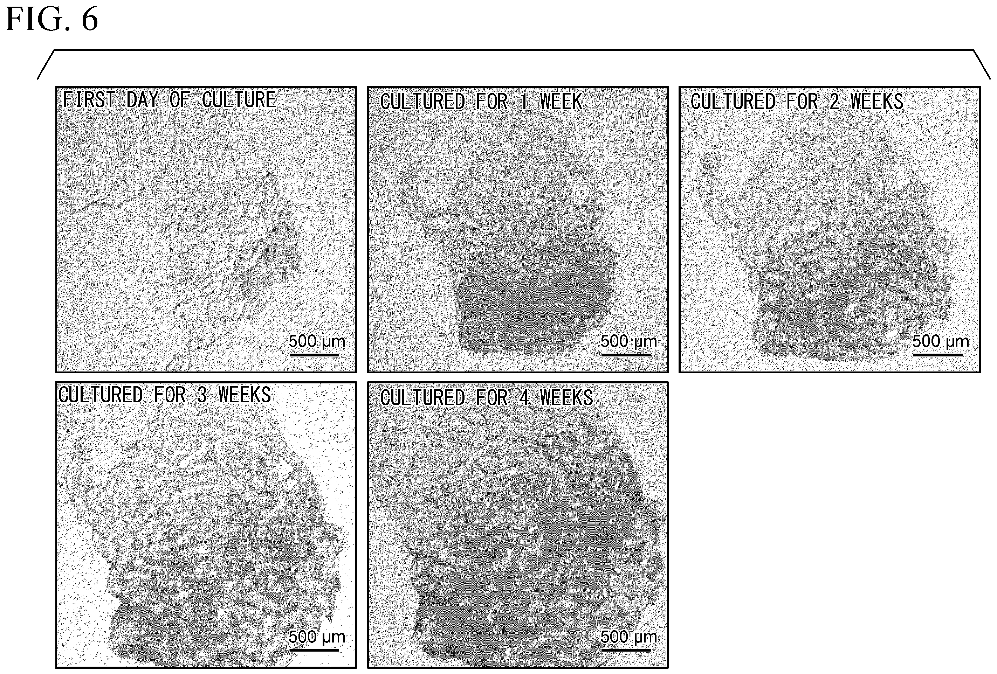

FIG. 5 is a diagram showing results of a testicular tissue culture experiment using a culture device 100 according to the comparative example. FIG. 6 is a diagram showing results of a testicular tissue culture experiment using a culture device 1 according to the first example.

In the present culture experiment, testicular tissues excised from mice at 7 days postpartum are used. As shown in FIGS. 5 and 6, it was confirmed that increase of the thickness of the seminiferous tubules of the testicular tissue was monitored as the culture progressed, and good culture was possible.

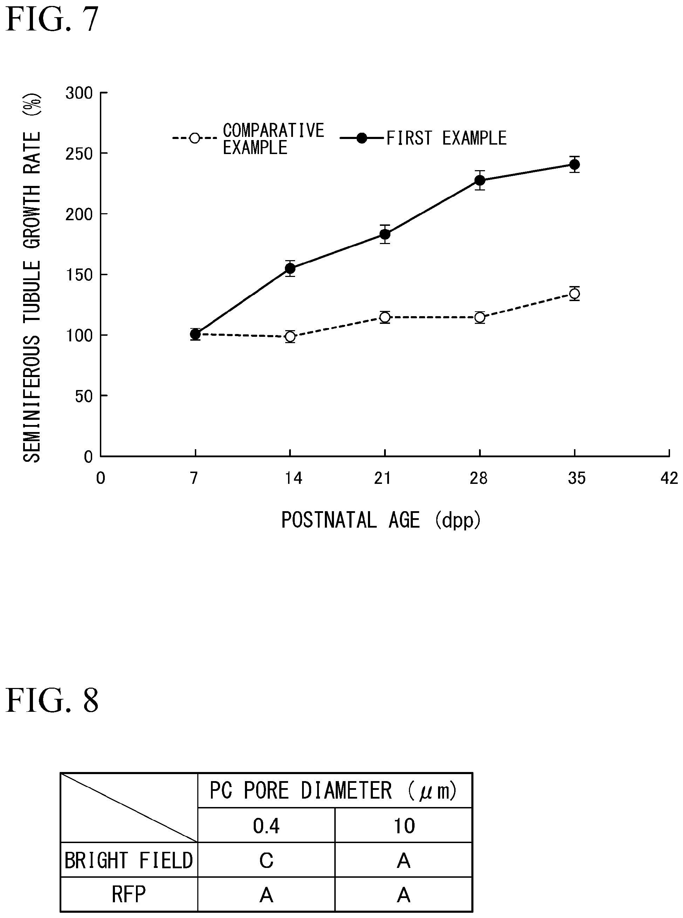

FIG. 7 is a graph showing postnatal age and seminiferous tubule growth rate of testicular tissues according to the first example and the comparative example. The seminiferous tubule growth rate is a value obtained by comparing the thicknesses of the fine tubular growth in the culture according to the first example and the comparative example. Specifically, the rate of change of the tube diameter of the seminiferous tubule was determined every week with the diameter of the seminiferous tubule on the first day of culture as 100%. As a result, as shown in FIG. 7, it was confirmed that the seminiferous tubules were thicker in a case where the culture device 1 according to the first example was used than in a case where the culture device 100 according to the comparative example was used. From this, it was shown that according to the culture device 1 according to the first example, it was possible to perform a culture equal to or higher than that of the organ culture method of the related art using the agarose gel 130.

Hereinafter, preferred aspects of the membrane member 22 will be described.

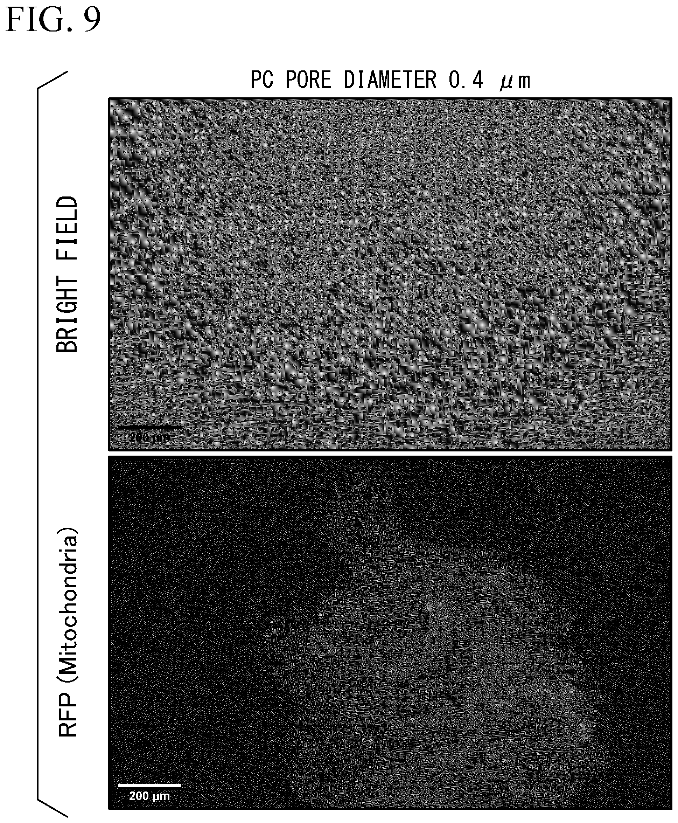

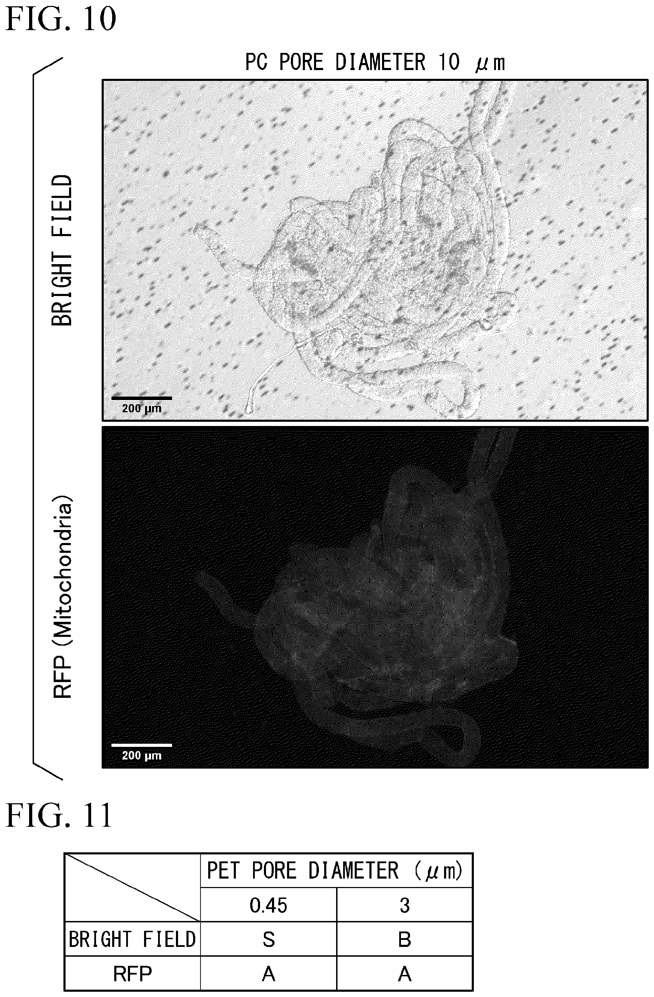

FIG. 8 is a table summarizing a relationship between a pore diameter and visibility of a culture subject 3 in a case where polycarbonate is used as the membrane member 22. FIG. 9 is a comparison photograph of a bright field and a fluorescence field (RFP) using an inverted microscope 30 in a case where polycarbonate having a pore diameter of 0.4 μm is used as a membrane member 22. FIG. 10 is a comparison photograph of a bright field and a fluorescence field (RFP) using an inverted microscope 30 in a case where polycarbonate having a pore diameter of 10 μm is used as a membrane member 22. "S", "A", "B", and "C" shown in FIGS. 8 and 10 indicate the monitorability (visibility) of the testicular tissue. Specifically, "S" is the most easily seen, and the easiness of seeing decreases in the order of "A", "B", and "C".

It is noted that in the fluorescence field monitoring, testicular tissues excised from gene-modified mice were used. In this mouse, since the mitochondria in the sperm cells are labeled with a red fluorescent protein (RFP), the spermatogenesis can be evaluated by fluorescence monitoring. It is noted that it is also possible to use testicular tissues of a gene-modified mouse in which acrosin in a sperm cell is labeled with a green fluorescent protein (GFP).

As shown in FIG. 9, in a case where polycarbonate having a pore diameter of 0.4 μm was used as the membrane member 22, the testicular tissue could hardly be monitored in the bright field, and the tissue could be monitored in the fluorescence field by RFP. Therefore, in this case, the visibility of the testicular tissue is "C" in the bright field and "A" in the RFP (see FIG. 8).

On the other hand, as shown in FIG. 10, in a case where polycarbonate having a pore diameter of 10 μm was used as the membrane member 22, the testicular tissue could be monitored in the bright field and the fluorescence field of RFP. Therefore, in this case, the visibility of the testicular tissue is "A" in the bright field and "A" in the RFP (see FIG. 8). That is, in a case where the material of the membrane member 22 is polycarbonate, the pore diameter is preferably 10 μm or more from the viewpoint of visibility.

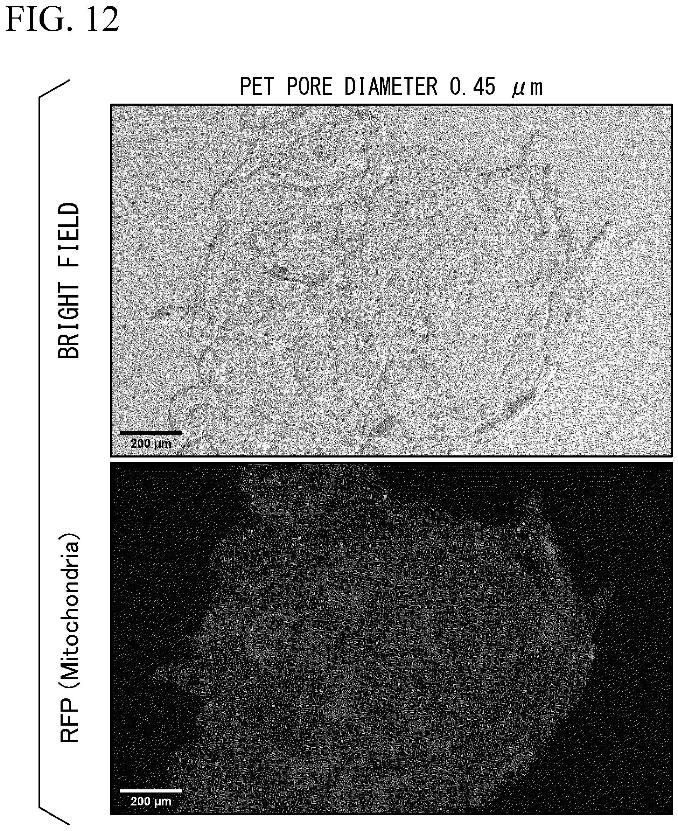

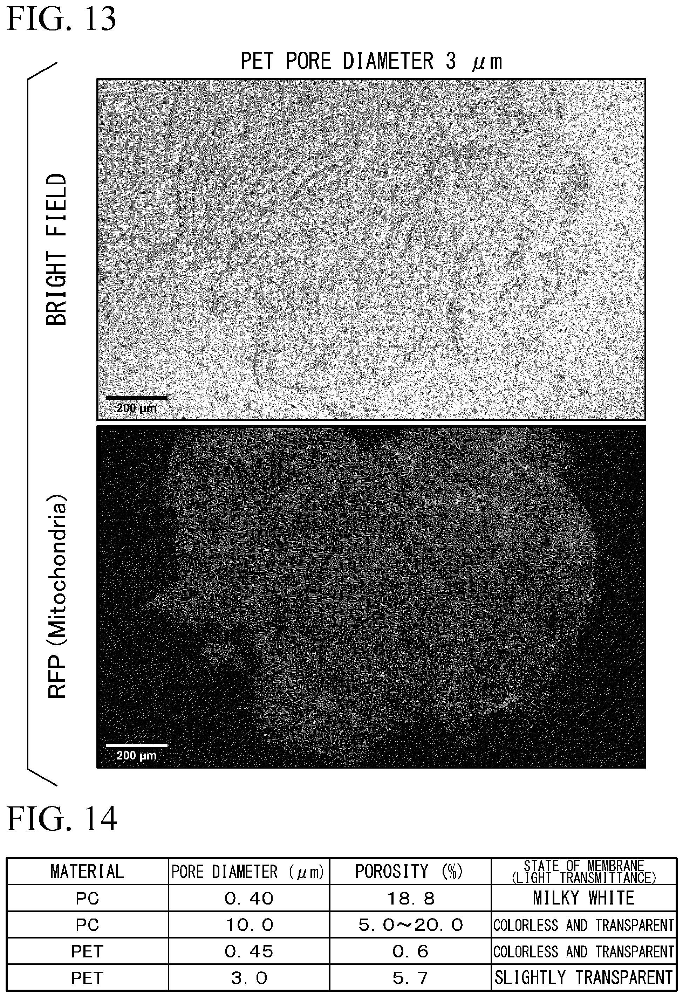

FIG. 11 is a table summarizing a relationship between a pore diameter and visibility of a culture subject 3 in a case where polyethylene terephthalate is used as the membrane member 22. FIG. 12 is a comparison photograph of a bright field and a fluorescence field (RFP) using an inverted microscope 30 in a case where polyethylene terephthalate having a pore diameter of 0.45 μm is used as a membrane member 22. FIG. 13 is a comparison photograph of a bright field and a fluorescence field (RFP) using an inverted microscope 30 in a case where polyethylene terephthalate having a pore diameter of 3 μm is used as a membrane member 22.

As shown in FIG. 12, in a case where polyethylene terephthalate having a pore diameter of 0.45 μm was used as the membrane member 22, the testicular tissue could be clearly monitored in the bright field and the fluorescence field of RFP. Therefore, in this case, the visibility of the testicular tissue is "S" in the bright field and "A" in the RFP (see FIG. 11).

On the other hand, as shown in FIG. 13, in a case where polyethylene terephthalate having a pore diameter of 3 $\mu$m was used as the membrane member 22, the testicular tissue could be monitored in the bright field and the fluorescence field of RFP, but a large number of pores were reflected. Therefore, in this case, the visibility of the testicular tissue is "B" in the bright field and "A" in the RFP (see FIG. 11). From the above, in a case where the material of the membrane member 22 is polyethylene terephthalate, the pore diameter is preferably 0.45 $\mu$m from the viewpoint of visibility.

FIG. 14 is a table summarizing the materials, pore diameters, porosities, and light transmittances of the membrane members 22 used in the first example. In a case of performing monitoring in bright field with transmitted light using the inverted microscope 30, the light transmittance of the membrane member 22 varies depending on the pore diameter and the porosity of the membrane member 22 used. As shown in FIG. 14, the membrane member 22 made of polycarbonate having a pore diameter of 0.4 $\mu$m is cloudy, and sufficient light transmittance required for optical monitoring cannot be obtained. The membrane member 22 of polyethylene terephthalate having a pore diameter of 3 $\mu$m is slightly whitish, and it is not impossible to perform optical monitoring, but it is not optimal. On the other hand, the other membrane member 22 (polycarbonate having a pore diameter of 10 $\mu$m and polyethylene terephthalate having a pore diameter of 0.45 $\mu$m) are colorless and transparent and have sufficient light transmittance.

In a case of monitoring the membrane member 22 with RFP, which is fluorescence monitoring with reflected light using an inverted microscope 30, since excitation light for monitoring is irradiated from the bottom wall portion 12 side, the visibility is uniformly "A" regardless of the light transmittance of the membrane member 22 and the pore diameter of 0.4 $\mu$m to 10 $\mu$m. In a case of monitoring in a bright field, since light for monitoring is irradiated from the membrane member 22 side, the light transmittance of the membrane member 22 has a large effect, and thus the visibility is "A" or "S" in the membrane member 22 of polycarbonate having a pore diameter of 10 $\mu$m and polyethylene terephthalate having a pore diameter of 0.45 $\mu$m, in which the light transmittance is sufficiently obtained. It is noted that regardless of the pore diameter, the porosity, the light transmittance, and the like of the membrane member 22, since the culture subject 3 can be sufficiently cultured, it is only required to select the membrane member 22 having excellent light transmittance in a case of monitoring in a bright field.

(Second embodiment)

Next, a second embodiment of the present invention will be described. In the following description, the same or equivalent configurations as those in the above-described embodiment are denoted by the same reference numerals, and descriptions thereof will be simplified or omitted.

The culture device 1 according to the second embodiment is different from the above-described embodiment in that the material of the bottom wall portion 12 shown in FIG. 1 is a 4-methyl-1-pentene-based polymer.

The 4-methyl-1-pentene-based polymer has light transmittance and gas permeability as in the above-described polydimethylsiloxane (PDMS), but is superior to the polydimethylsiloxane in the following points. First, the 4-methyl-1-pentene-based polymer is harder than the polydimethylsiloxane, and thus the shape of the bottom wall portion 12 is likely to be maintained. In addition, the 4-methyl-1-pentene-based polymer has lower adsorptivity for a drug or a culture solution component than polydimethylsiloxane, and appropriately permeates oxygen. Therefore, the 4-methyl-1-pentene-based polymer is suitable as a material of the bottom wall portion 12 of the culture device 1. In a case where the oxygen permeability of the bottom wall portion 12 is too low, the cell tissue of the culture subject 3 does not grow sufficiently, and in a case where the oxygen permeability is too high, the cell tissue of the culture subject 3 is enlarged and the cells are crushed in the culture room 4. Therefore, the bottom wall portion 12 may appropriately permeate oxygen. Specifically, the oxygen permeability of the bottom wall portion 12 at a temperature of 23°C and a humidity of 0% is 4,500 to 90,000 $cm^3/(m^2 \times 24\,h \times atm)$, preferably 4,500 to 67,500 $cm^3/(m^2 \times 24\,h \times atm)$, more preferably 4,500 to 47,000 $cm^3/(m^2 \times 24\,h \times atm)$, and still more preferably 4,500 to 45,000 $cm^3/(m^2 \times 24\,h \times atm)$. Although the detailed reason is not clear, by using the 4-methyl-1-pentene-based polymer as the material of the bottom wall portion 12, the culture function of the culture device 1 is improved as compared with a case where other gas-permeable materials such as polydimethylsiloxane and a tetrafluoroethylene-hexafluoropropylene copolymer (FEP) are used, and the culture subject 3 (particularly, the testicular tissue) can be favorably cultured.

In the present embodiment, the 4-methyl-1-pentene homopolymer and the copolymer of 4-methyl-1-pentene and another monomer are collectively referred to as a "4-methyl-1-pentene-based polymer". The copolymer of 4-methyl-1-pentene and another monomer, which is an example of the 4-methyl-1-pentene-based polymer, may be any of a random copolymer, an alternating copolymer, a block copolymer, or a graft copolymer. As the copolymer of 4-methyl-1-pentene and another monomer, a copolymer of 4-methyl-1-pentene and at least one olefin selected from ethylene or $\alpha$-olefin having 3 to 20 carbon atoms (excluding 4-methyl-1-pentene) is preferable since the copolymer has high strength, is less likely to be torn or broken even in a case of being used as a material of the bottom wall portion 12, and has less deflection.

The 4-methyl-1-pentene-based polymer is preferably at least one polymer selected from a 4-methyl-1-pentene homopolymer or a copolymer of 4-methyl-1-pentene and at least one olefin selected from ethylene or an α-olefin having 3 to 20 carbon atoms (excluding 4-methyl-1-pentene), and more preferably a copolymer of 4-methyl-1-pentene and at least one olefin selected from ethylene or an α-olefin having 3 to 20 carbon atoms (excluding 4-methyl-1-pentene).

Examples of the olefin include ethylene, propylene, 1-butene, 1-hexene, 1-heptene, 1-octene, 1-decene, 1-tetradecene, 1-hexadecene, 1-heptadecene, 1-octadecene, and 1-eicosene. The olefin can be appropriately selected depending on the physical properties required for the culture member. For example, from the viewpoints of appropriate oxygen permeability and excellent rigidity, the olefin is preferably an α-olefin having 8 to 18 carbon atoms, and more preferably at least one selected from 1-octene, 1-decene, 1-dodecene, 1-tetradecene, 1-hexadecene, 1-heptadecene, or 1-octadecene. In a case where the number of carbon atoms in the olefin is in the above-described range, the processability of the polymer is further improved, and the appearance defect of the bottom wall portion 12 due to cracks or breakage of end portions tends to be less likely to occur. In addition, the defective product generation rate of the bottom wall portion 12 is reduced.

The olefin can be used alone or in combination of two or more types thereof. From the viewpoint of the strength of the material, the number of carbon atoms is preferably 2 or more and more preferably 10 or more. In a case where two or more different types of α-olefins are combined, it is particularly preferable to combine at least one selected from 1-tetradecene or 1-hexadecene and at least one selected from 1-heptadecene or 1-octadecene.

The amount of a constitutional unit derived from 4-methyl-1-pentene in the 4-methyl-1-pentene-based polymer is preferably 60 to 100 mol% and more preferably 80 to 98 mol%.

In addition, in a case where the 4-methyl-1-pentene-based polymer is a copolymer of 4-methyl-1-pentene and at least one olefin selected from ethylene or an α-olefin having 3 to 20 carbon atoms (excluding 4-methyl-1-pentene), the amount of the constitutional unit derived from the at least one olefin selected from ethylene or an α-olefin having 3 to 20 carbon atoms (excluding 4-methyl-1-pentene) in the copolymer is preferably 0 to 40 mol% and more preferably 2 to 20 mol%. The amount of these constitutional units is set such that the total amount of repeating constitutional units in the 4-methyl-1-pentene-based polymer is 100 mol%. In a case where the amount of the constitutional unit is within the above-described range, a bottom wall portion 12 having excellent processability and uniformity is obtained, and since the balance between the toughness and the strength of the film is good, the deflection is also small.

The 4-methyl-1-pentene-based polymer may have a constitutional unit (hereinafter, also referred to as "other constitutional units") other than the constitutional unit derived from 4-methyl-1-pentene and the constitutional unit derived from ethylene and α-olefin having 3 to 20 carbon atoms, as long as the effect of the present invention is not impaired. The amount of the other constitutional units is, for example, 0 to 10.0 mol%. In a case where the 4-methyl-1-pentene-based polymer has other constitutional units, the other constitutional units may be one type or two or more types.

Examples of the monomer from which the other constitutional units are derived include a cyclic olefin, an aromatic vinyl compound, a conjugated diene, a non-conjugated polyene, a functional vinyl compound, a hydroxyl group-containing olefin, and a halogenated olefin. As the cyclic olefin, the aromatic vinyl compound, the conjugated diene, the non-conjugated polyene, the functional vinyl compound, the hydroxyl group-containing olefin, and the halogenated olefin, for example, the compounds described in paragraphs [0035] to [0041] of JP2013-169685A can be used.

The 4-methyl-1-pentene-based polymer may be used alone or in combination of two or more types thereof.

A commercially available product can also be used as the 4-methyl-1-pentene-based polymer. Specific examples thereof include TPX MX001, MX002, MX004, MX0020, MX021, MX321, RT18, RT31, and DX845 (all of which are trademarks) manufactured by Mitsui Chemicals, Inc. In addition, a 4-methyl-1-pentene-based polymer manufactured by other manufacturers can also be preferably used as long as it satisfies the above-described requirements. These commercially available products may be used alone or in combination of two or more types thereof.

The 4-methyl-1-pentene-based polymer generally has a melting point of 200°C to 240°C and has high heat resistance. In addition, since hydrolysis does not occur and water resistance, boiling water resistance, and steam resistance are excellent, the culture device having the bottom wall portion containing the 4-methyl-1-pentene-based polymer can be subjected to high-pressure steam sterilization treatment. Since the 4-methyl-1-pentene-based polymer also has a high visible light transmittance (usually 90% or more) and does not emit autofluorescence, the culture subject 3 can be easily monitored in the culture device 1 having the bottom wall portion 12 containing the 4-methyl-1-pentene-based polymer. Furthermore, since the polymer exhibits excellent chemical resistance to most chemicals and is difficult to sorb a drug, the polymer is suitably used for drug discovery screening applications and diagnostic applications. The 4-methyl-1-pentene-based polymer can be heat-sealed, and not only thermal fusion welding between the materials but also thermal adhesion to other materials are easily performed. In addition, since the polymer can be thermally molded, it is easy to mold the polymer into any shape, and for example, it is easy to mold the polymer by an imprinting method or an insert method.

The weight-average molecular weight (Mw) of the 4-methyl-1-pentene-based polymer, which is measured by gel permeation chromatography (GPC) using standard polystyrene as a reference substance, is preferably 10,000 to 2,000,000, more preferably 20,000 to 1,000,000, and still more preferably 30,000 to 500,000. Here, the concentration of the sample in the GPC measurement can be, for example, 1.0 to 5.0 mg/ml. In addition, the molecular weight distribution (Mw/Mn) of the 4-methyl-1-pentene-based polymer is preferably 1.0 to 30, more preferably 1.1 to 25, and still more preferably 1.1 to 20. As the solvent used in GPC, orthodichlorobenzene is preferably used. In addition, examples of the measurement conditions include the following conditions, but the measurement conditions are not limited thereto.

<Measurement conditions of weight-average molecular weight (Mw) and molecular weight distribution (Mw/Mn)>

The weight-average molecular weight (Mw) and the number-average molecular weight (Mn) of the polymer (4-methyl-1-pentene-based polymer) dissolved in the orthodichlorobenzene are measured by calibrating the molecular weight with respect to the standard polystyrene under the following conditions.

· Apparatus: gel permeation chromatograph HLC-8321 GPC/HT type (manufactured by TOSOH CORPORATION)
· Data analysis software: Empower 3 (manufactured by Waters Corporation)
· Detector: differential refractometer
· Series-connected columns: TSKgel GMH6-HT (2 columns) and TSKgel GMH6-HTL (2 columns)
· Column temperature: 140°C
· Flow rate: 1.0 ml/min
· Sample concentration: 1.5 mg/ml

By setting the weight-average molecular weight (Mw) to be equal to or less than the above-described upper limit, in a case of molding the 4-methyl-1-pentene-based polymer, it is easy to suppress the occurrence of defects such as gel in a film produced by melt molding, and it is easy to form a film having a uniform surface. In addition, in a case of being produced by a solution casting method, solubility in a solvent is further improved, it is easy to suppress defects such as gel in the film, and it is easy to form a film having a uniform surface.

In addition, in a case where the weight-average molecular weight (Mw) is set to be equal to or more than the above-described lower limit, the bottom wall portion 12 tends to have sufficient strength. Furthermore, by setting the molecular weight distribution within the above-described range, it is easy to suppress stickiness on the surface of the bottom wall portion 12, and the toughness tends to be sufficient, and it is easy to suppress the occurrence of bending, cracking, and the like.

In a case where two or more types of 4-methyl-1-pentene-based polymers are used, the weight-average molecular weight (Mw) and the molecular weight distribution (Mw/Mn) of each of the 4-methyl-1-pentene-based polymers may be within the above-described ranges.

Since the 4-methyl-1-pentene-based polymer has the above-described excellent characteristics, the culture device 1 in which at least the bottom wall portion 12 is formed of the 4-methyl-1-pentene-based polymer does not adversely affect the culture, has excellent stability and light transmittance, and can be subjected to a sterilization treatment, which is extremely excellent.

The method for manufacturing the 4-methyl-1-pentene-based polymer may be any method as long as 4-methyl-1-pentene, an olefin, and other monomers can be polymerized. In addition, a chain transfer agent, for example, hydrogen may be coexisting to control the molecular weight or the molecular weight distribution. The equipment used for the manufacture is also not limited. The polymerization method may be a known method, and may be a vapor phase method, a slurry method, a solution method, or a bulk method. The slurry method or the solution method is preferable. In addition, the polymerization method is a single-stage polymerization method or a multiphase polymerization method such as a two-phase polymerization method, and may be a method of blending a plurality of polymers having different molecular weights in a polymerization system. In any of the single-stage or multi-stage polymerization methods, in a case where hydrogen is used as a chain transfer agent, the hydrogen may be added all at once or may be added in a divided manner, for example, at the beginning, middle, or end of the polymerization. The polymerization may be performed at room temperature, and may be performed by heating as necessary, but from the viewpoint of the efficiency of the polymerization, the polymerization is preferably performed at 20°C to 80°C and particularly preferably performed at 40°C to 60°C. The catalyst used for the manufacture is also not limited, but from the viewpoint of polymerization efficiency, it is preferable to use, for example, a solid titanium catalyst component (I) described in WO2006/054613A or an olefin polymerization catalyst (metallocene catalyst) containing a transition metal compound (A) described in WO2014/050817A.

It is noted that in a case where the bottom wall portion 12 is formed of a composition containing a 4-methyl-1-pentene-based polymer, the amount of the 4-methyl-1-pentene-based polymer in 100% by mass of the composition forming the bottom wall portion 12 is preferably 90% by mass or more and less than 100% by mass, more preferably 95% by mass or more and less than 100% by mass, and particularly preferably 99% by mass or more and less than 100% by mass. In a case where a large amount of components other than the 4-methyl-1-pentene-based polymer are contained, not only a decrease in oxygen permeability but also a decrease in transparency or a decrease in strength are caused.

The material forming the bottom wall portion 12 may include a component other than the 4-methyl-1-pentene-based polymer. Examples of the components other than the 4-methyl-1-pentene-based polymer include additives such as a heat-resistant stabilizer, a light-resistant stabilizer, a processing aid, a plasticizer, an antioxidant, a lubricant, an antifoaming agent, an antiblocking agent, a colorant, a modifier, an antibacterial agent, an antifungal agent, and an anti-fogging agent.

[Second example]

Hereinafter, a second example of the present invention will be described. The present invention is not limited to the following second example as in the first example, and can be appropriately modified and implemented within the scope not departing from the gist thereof.

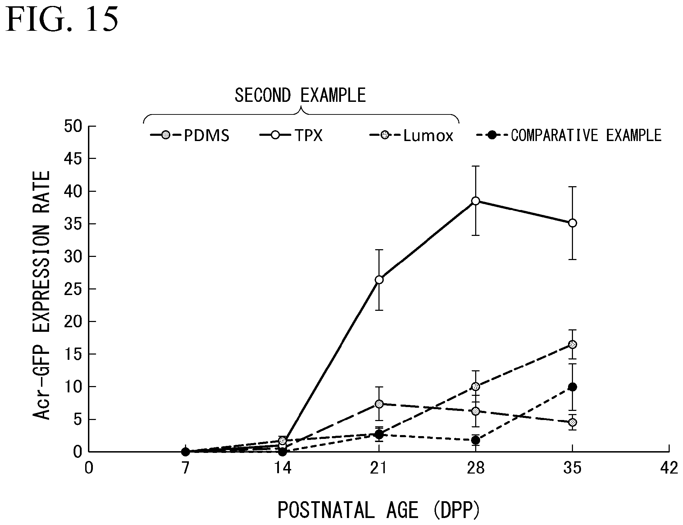

FIG. 15 is a graph showing postnatal age and Acr-GFP expression rate of testicular tissues according to a second example and the comparative example. The Acr-GFP expression rate is obtained by the following expression (1) in a case where the fluorescence field area of the testicular tissue of the gene-modified mouse in which the acrosin in the sperm cell is labeled with green fluorescent protein (GFP) is defined as A1 and the bright field area of the testicular tissue is defined as A2.

$$\text{Acr-GFP expression rate } (\%) = A1/A2 \ ... \ (1)$$

In the second example, the height of the testicular tissue was controlled to 85 $\mu$m using the culture chip 20. In addition, in the second example, polydimethylsiloxane (PDMS), TPX (registered trademark) (manufactured by Mitsui Chemicals, Inc., molecular weight (Mw) = 428,000, molecular weight distribution (Mw/Mn) = 4.1) which is a 4-methyl-1-pentene-based polymer, and LUMOX (registered trademark) (manufactured by SARSTEDT AG & Co., KG) which is a tetrafluoroethylene-hexafluoropropylene copolymer (FEP) were used as materials of the bottom wall portion 12. In addition, as a comparative example, a culture device 100 shown in FIG. 4 was used.

The graph shown in FIG. 15 shows experimental results in a case where the thickness of the bottom wall portion 12 is 500 $\mu$m and the number of samples is 6, and in a case of polydimethylsiloxane (PDMS). In addition, in a case of a 4-methyl-1-pentene-based polymer (TPX), the experimental results in a case where the thickness of the bottom wall portion 12 is 50 $\mu$m, the oxygen permeability at a temperature of 23°C and a humidity of 0% is 38,240 cm$^3$/(m$^2$ $\times$ 24 h $\times$ atm), and the number of samples is 12 are shown. It is noted that in the case of TPX, the thickness of the bottom wall portion 12 is 1/10 with respect to the polydimethylsiloxane, but since the oxygen permeability of TPX is approximately 1/10 with respect to the polydimethylsiloxane, the conditions are approximately the same. In addition, in the case of Lumox, the experimental results in a case where the thickness of the bottom wall portion 12 is set to 25 $\mu$m and the number of samples is set to 2 are shown. In the case of LUMOX (registered trademark), the conditions are substantially the same in consideration of the relationship between the bottom wall portion 12 and the oxygen permeability. As shown in FIG. 15, it can be seen that in the case where the material of the bottom wall portion 12 is a 4-methyl-1-pentene-based polymer (TPX), the Acr-GFP expression rate is excellent compared with the case where the material of the bottom wall portion 12 is polydimethylsiloxane (PDMS) or Lumox, and the comparative example.

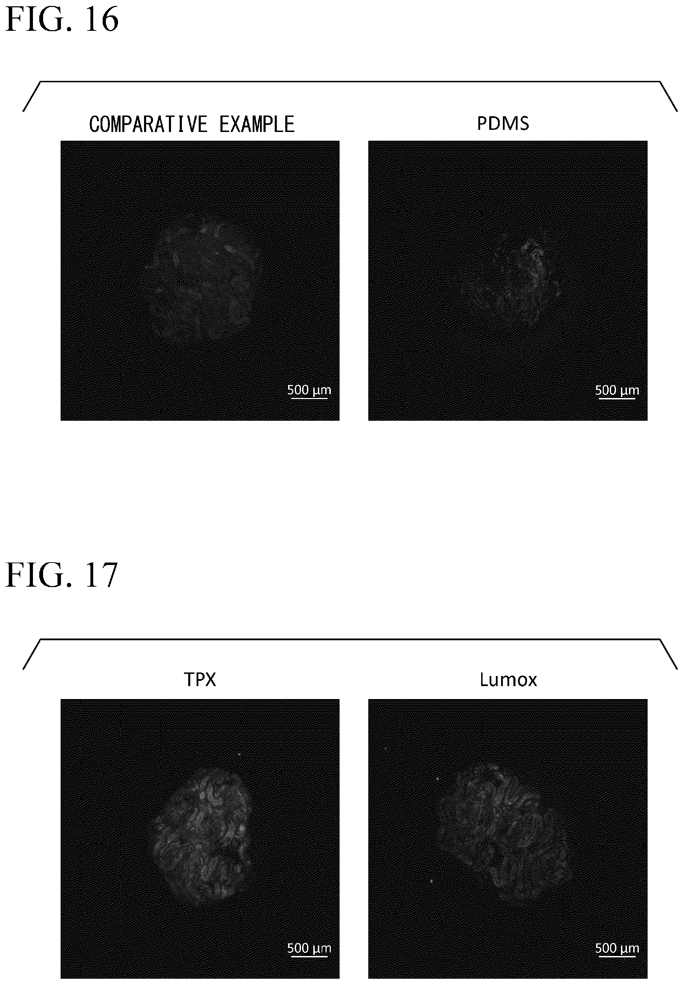

FIG. 16 is a diagram comparing fluorescence images of testicular tissues at the postnatal age (35th day) in a case where polydimethylsiloxane (PDMS) is used as a bottom wall portion 12, with Comparative Example. FIG. 17 is a diagram comparing fluorescence images of testicular tissues at postnatal age (35th day) in a case where a 4-methyl-1-pentene-based polymer (TPX) and LumoX (registered trademark) are used as a bottom wall portion 12. As shown in FIGS. 16 and 17, on the postnatal age day (35th day), in a case where a 4-methyl-1-pentene-based polymer (TPX) was used as the bottom wall portion 12, it was confirmed that Acr-GFP was clearly expressed in the testicular tissue as compared with other materials or the comparative example. From this, it was shown that in a case where the 4-methyl-1-pentene-based polymer (TPX) was used as the bottom wall portion 12 in the culture device 1 according to the second example, the expression of Acr-GFP was excellent and the fluorescence monitoring was easy, as compared with the case where the 4-methyl-1-pentene-based polymer (TPX) and Lumox were used or the comparative example.

(Third embodiment)

Next, a third embodiment of the present invention will be described. In the following description, the same or equivalent configurations as those in the above-described embodiment are denoted by the same reference numerals, and descriptions thereof will be simplified or omitted.

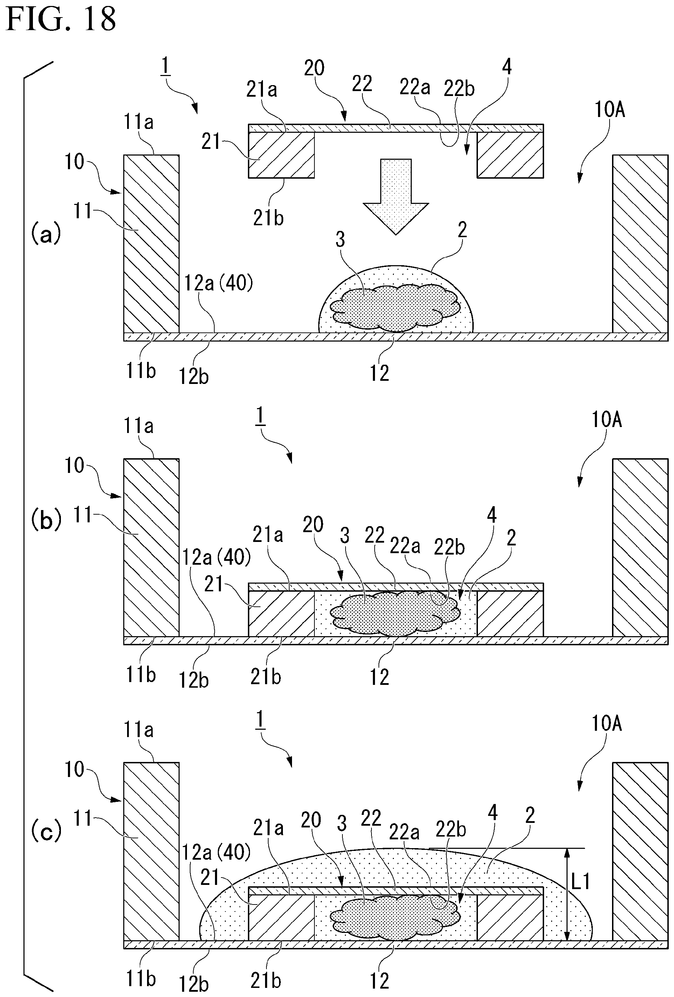

FIG. 18 is a diagram showing a state in which a culture solution 2 and a culture subject 3 are set in a culture device 1 according to a third embodiment. In the culture device 1 according to the third embodiment, the upper surface 12a of the bottom wall portion 12 has hydrophobicity. For example, in a case where the material of the bottom wall portion 12 is a 4-methyl-1-pentene-based polymer, the bottom wall portion 12 itself has hydrophobicity. That is, the entire upper surface 12a of the bottom wall portion 12 is the hydrophobic region 40. It is noted that the term "hydrophobic" as used herein means to have a parameter that the upper surface 12a of the bottom wall portion 12 has a water contact angle of 80° or more and 160° or less, preferably 90° or more and 160° or less, more preferably more than 100° and 150° or less, and still more preferably 105° or more and 130° or less.

It is noted that the method of measuring a water contact angle is not particularly limited, and a known method can be used, but the method is preferably a sessile drop method. The water contact angle can be measured, for example, according to Japanese Industrial Standards JIS-R3257 (method for testing wettability of surface of substrate glass), by a method of dropping a water droplet having a capacity of 4 μL or less, which is regarded as a sphere under a constant temperature and humidity condition of 25 ± 5°C and 50 ± 10%, on the surface of a measurement sample produced using a culture member or the same material as the culture member, and measuring the angle of the contact interface between the measurement sample surface and the water droplet within 1 minute after the water droplet comes into contact with the measurement sample surface by the sessile drop method.

(a) of FIG. 18 shows a first step of forming droplets by dropping a culture solution 2 onto an upper surface 12a of a bottom wall portion 12 of a container main body 10 having the bottom wall portion 12 having gas permeability. In addition, (a) of FIG. 18 shows a second step of enclosing the culture subject 3 in the droplet. According to the above-described configuration, as shown in (a) of FIG. 18, by dropping the culture solution 2 onto the upper surface 12a of the bottom wall portion 12, it is easy to produce droplets (tiny water droplets on a micrometer scale). As a result, the culture subject 3 is easily enclosed in the culture solution 2.

(b) of FIG. 18 shows a third step of forming a culture room 4 that accommodates the culture subject 3 with the bottom wall portion 12, a frame portion 21, and a membrane member 22 by installing the frame portion 21 that abuts on the upper surface 12a of the bottom wall portion 12 and surrounds the droplets in which the culture subject 3 is enclosed, and a membrane member 22 that has liquid permeability or liquid-containing substance permeability and is fixed to the upper surface 21a of the frame portion 21. In a case where the culture chip 20 is installed on the bottom wall portion 12 by forming the droplets of the culture solution 2 as described above, the culture room 4 is easily filled with the culture solution 2.

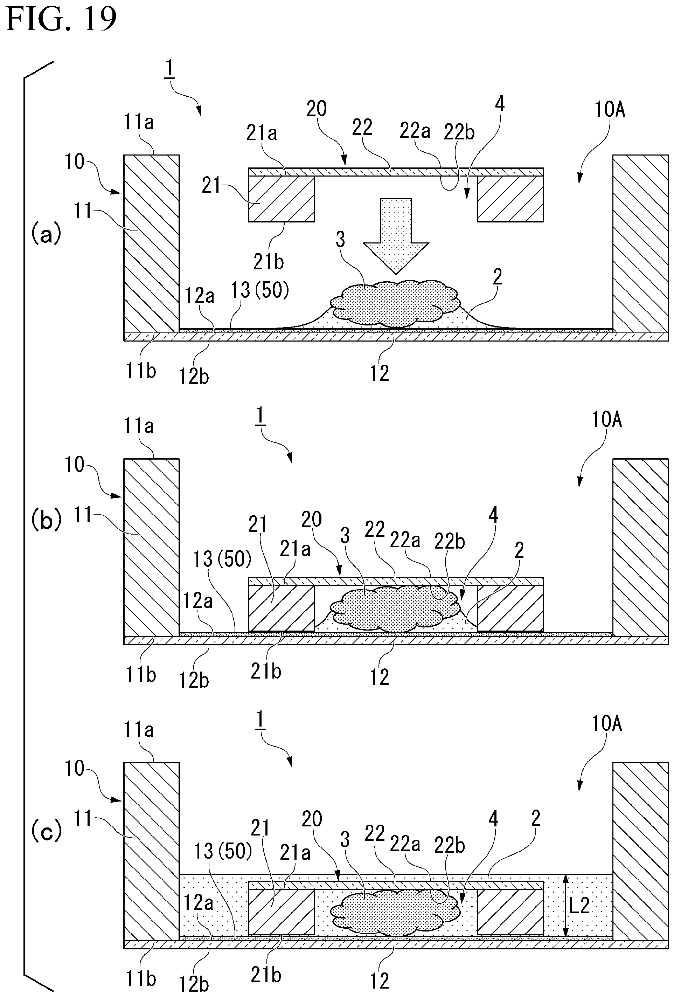

FIG. 19 is a diagram showing a state in which a culture solution 2 and a culture subject 3 are set in a culture device 1 in which an upper surface 12a of a bottom wall portion 12 is hydrophilic. The upper surface 12a of the bottom wall portion 12 shown in (a) of FIG. 19 is covered with a hydrophilic coating layer 13. That is, the entire upper surface 12a of the bottom wall portion 12 is the hydrophilic region 50. The "hydrophilicity" means that the material has a parameter other than the "hydrophobicity" described above.

With this configuration, as shown in (a) of FIG.

19, even in a case where the culture solution 2 is dropped onto the upper surface 12a of the bottom wall portion 12, the culture solution 2 is wetted and spread, and droplets cannot be formed. In addition, as shown in (b) of FIG. 19, in a case where the culture chip 20 is installed on the bottom wall portion 12, air is likely to remain in the culture room 4. Furthermore, the culture solution 2 enters the lower surface 21b of the frame portion 21, and it is difficult to install (adhere) the culture chip 20 to the bottom wall portion 12.

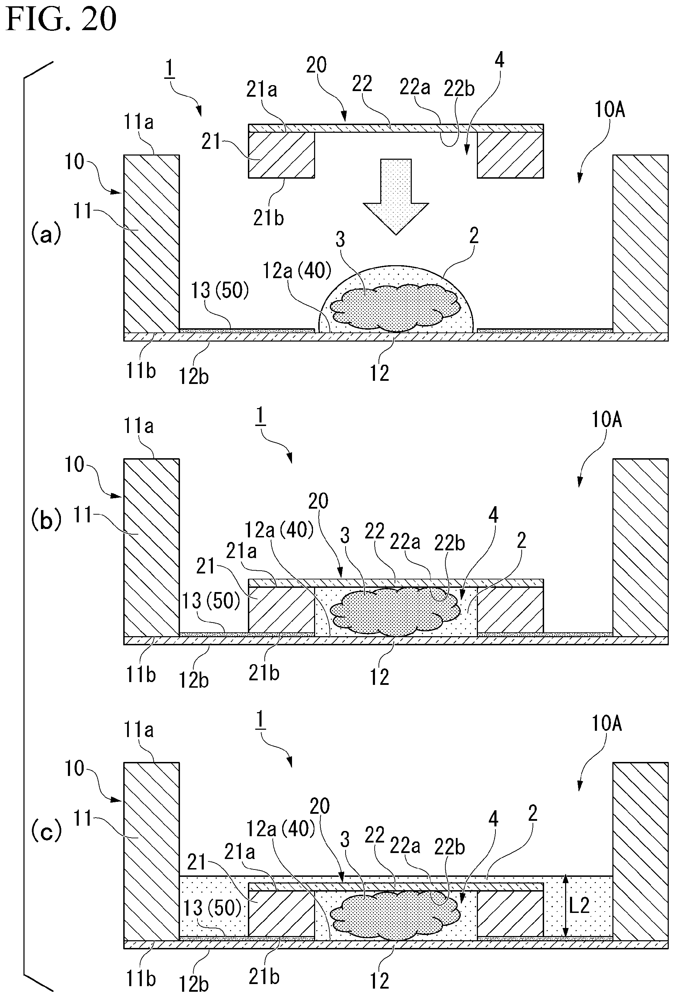

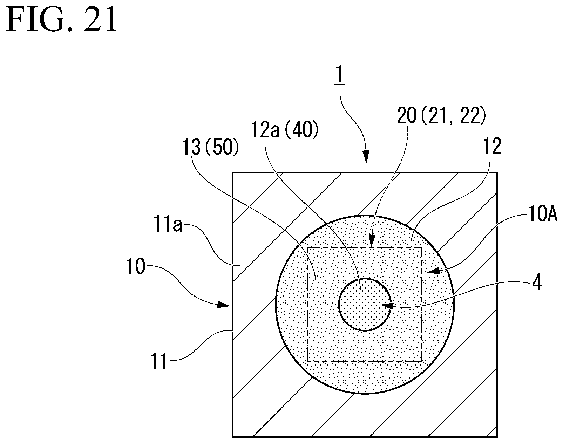

On the other hand, in a case where the culture solution 2 is supplied to the culture solution accommodation chamber 10A, the supply amount of the culture solution 2 is smaller in a case where the upper surface 12a of the bottom wall portion 12 is made hydrophilic than in a case where the upper surface 12a of the bottom wall portion 12 is made hydrophobic. Specifically, in a case where the upper surface 12a of the bottom wall portion 12 is made hydrophobic, a liquid level L1 is required to immerse the culture chip 20 in the culture solution 2, as shown in (c) of FIG. 18. On the other hand, in a case where the upper surface 12a of the bottom wall portion 12 is made hydrophilic, as shown in (c) of FIG. 19, the liquid level L2 lower than the liquid level L1 is sufficient for immersing the culture chip 20 in the culture solution 2. Therefore, the configurations shown in FIGS. 20 and 21 may be employed.

FIG. 20 is a diagram showing a state in which a culture solution 2 and a culture subject 3 are set in a culture device 1 according to a modification example of the third embodiment. FIG. 21 is a plan view of the container main body 10 shown in FIG. 20. As shown in these drawings, in the bottom wall portion 12, a portion of the upper surface 12a that forms the culture room 4 has hydrophobicity (hydrophobic region 40), and a portion other than the portion that forms the culture room 4 has hydrophilicity (hydrophilic region 50).

According to the above-described configuration, as shown in (a) of FIG. 20, by dropping the culture solution 2 into the hydrophobic region 40 of the bottom wall portion 12, it is easy to produce droplets, and it is easy to enclose the culture subject 3 in the droplets. In addition, as shown in (b) of FIG. 20, in a case where the culture chip 20 is installed on the bottom wall portion 12, the culture room 4 is easily to be filled with the culture solution 2. Furthermore, since the culture solution 2 is not wetted and spread, it is easy to install the culture chip 20 on the bottom wall portion 12. In addition, as shown in (c) of FIG. 20, the liquid level L2 is sufficient for immersing the culture chip 20 in the culture solution 2. As a result, since sufficient cell culture can be performed with a small amount of the culture solution 2, the running cost is reduced.

In addition, as shown in FIG. 21, the culture chip 20 (the frame portion 21 and the membrane member 22) has a rectangular outer shape, but the inner shape of the frame portion 21 forming the culture room 4 may be circular. In a case where the inner shape of the frame portion 21 is circular, air is less likely to remain in the corner portion as compared with a case where the inner shape of the frame portion 21 is quadrangular, and the culture room 4 is easily filled with the culture solution 2 in a case where the culture chip 20 is installed. In a case where the outer shape of the culture chip 20 is rectangular, mass production is possible by cutting a sheet or the like, and the culture chip 20 can be manufactured at a low cost.

It is noted that in another modification example of the third embodiment, a part or whole of the upper surface 12a of the bottom wall portion 12 may be subjected to a surface treatment or may be coated with a coating layer different from the hydrophilic coating layer 13 described above to form the specific region. The water contact angle of the specific region formed on the upper surface 12a of the bottom wall portion 12 can be, for example, 30° or more and 160° or less.

While preferred embodiments of the present invention have been described and illustrated above, it should be understood that these are exemplary of the present invention and should not be considered as limiting. Additions, omissions, substitutions, and other changes can be made without departing from the scope of the present invention. Accordingly, the present invention should not be considered as being limited by the foregoing description, and is only limited by the scope of the appended claims.

[Appendixes]

It is noted that the above-described culture device 1 is excellent not only in the method for monitoring a culture subject but also in the culture method of the culture subject 3 (for example, see FIG. 15). In a case of intending for the culture of the culture subject 3, the bottom wall portion 12 does not necessarily have light transmittance.

Therefore, a part or all of the above-described embodiments can be described as follows, but the present invention is not limited thereto, with the culture of the culture subject 3, in particular, the culture of the testicular tissue as the object and the purpose.

(Appendix 1)

A culture device including:

a container main body that accommodates a culture solution; and
a culture chip that forms a culture room inside the container main body,
wherein the container main body has a bottom wall portion having gas permeability, and
the culture chip has a frame portion that abuts on an upper surface of the bottom wall portion, and a membrane member that has liquid permeability or liquid-containing substance permeability, is fixed to an upper surface of the frame portion, and forms the culture room with the bottom wall portion and the frame portion.

(Appendix 2)

The culture device according to (Appendix 1), wherein a material of the bottom wall portion is a 4-methyl-1-pentene-based polymer.

(Appendix 3)

The culture device according to (Appendix 2), wherein the 4-methyl-1-pentene-based polymer is a copolymer of 4-methyl-1-pentene and at least one selected from ethylene or an $\alpha$-olefin having 3 to 20 carbon atoms (excluding 4-methyl-1-pentene).

(Appendix 4)

The culture device according to any one of (Appendix 1) to (Appendix 3),
wherein an oxygen permeability of the bottom wall portion at a temperature of 23°C and a humidity of 0% is 4,500 to 90,000 $cm^3/(m^2 \times 24\ h \times atm)$.

(Appendix 5)

The culture device according to any one of (Appendix 1) to (Appendix 4),
wherein the bottom wall portion has light transmittance.

(Appendix 6)

A cell culture method using the culture device according to any one of (Appendix 1) to (Appendix 5), in which the oxygen in a gas phase is supplied to a culture subject housed in the culture room through the bottom wall portion, and the culture solution is supplied to the culture subject through the membrane member.

(Appendix 7)

A cell culture method including:

a first step of adding dropwise a culture solution onto an upper surface of a bottom wall portion of a container main body having the bottom wall portion having gas permeability to form droplets;
a second step of enclosing a culture subject in the droplets; and
a third step of forming a culture room with the bottom wall portion and a frame portion for accommodating the culture subject by installing the frame portion that abuts on the upper surface of the bottom wall portion and surrounds the droplets in which the culture subject is enclosed, and a membrane member that has liquid permeability or liquid-containing substance permeability and is fixed to the upper surface of the frame portion.

Alternatively, a component in the above-described embodiment can be appropriately substituted with a well-known component within the scope not departing from the concept of the present invention, and the above-described modification examples may be appropriately combined with each other.

REFERENCE SIGNS LIST

1 Culture device
2 Culture solution
3 Culture subject
4 Culture room
10 Container main body
10A Culture solution accommodation chamber
11 Side wall portion
11a Upper surface
11b Lower surface
12 Bottom wall portion
12a Upper surface
20 Culture chip
21 Frame portion
21a Upper surface
21b Lower surface
22 Membrane member
22b Lower surface
30 Inverted microscope
40 Hydrophobic region
50 Hydrophilic region
100 Culture device
110 Container main body
110A Culture solution accommodation chamber
111 Side wall portion
112 Bottom wall portion
120 Culture chip
130 Agarose gel
H1 Height
L1 Liquid level
L2 Liquid level
W1 Width
W2 Width

**Claims**

1. A culture device comprising:

   a container main body that accommodates a culture solution; and
   a culture chip that forms a culture room inside the container main body,
   wherein the container main body has a bottom wall portion having light transmittance and gas permeability, and
   the culture chip has a frame portion that abuts on an upper surface of the bottom wall portion, and a membrane member that has liquid permeability or liquid-containing substance permeability, is fixed to an upper surface of the frame portion, and forms the culture room with the bottom wall portion and the frame portion.

2. The culture device according to Claim 1, wherein the oxygen in a gas phase is supplied to a culture subject housed in the culture room through the bottom wall portion, and the culture solution is supplied to the culture subject through the membrane member.

3. The culture device according to Claim 1, wherein a thickness of the culture subject housed in the culture room is controlled by a height of the frame portion.

4. The culture device according to Claim 1, wherein the membrane member has light transmittance.

5. The culture device according to Claim 1, wherein the frame portion is fixed to the upper surface of the bottom wall portion.

6. The culture device according to Claim 1, wherein the frame portion has a height within a range of 20 $\mu$m or more and 400 $\mu$m or less.

7. The culture device according to Claim 2,

   wherein the culture subject is a seminiferous tubule, and
   the frame portion has a height within a range of 50 $\mu$m or more and 350 $\mu$m or less.

8. The culture device according to Claim 2,

   wherein the culture subject is a testicular tissue of a mouse, and
   the frame portion has a height within a range of 50 $\mu$m or more and 200 $\mu$m or less.

9. The culture device according to Claim 1, wherein the upper surface of the bottom wall portion has hydrophobicity.

10. The culture device according to Claim 1, wherein a part of the upper surface of the bottom wall portion, that forms the culture room, has hydrophobicity, and a part other than the part that forms the culture room has hydrophilicity.

11. A method for monitoring a culture subject, the method comprising:
monitoring the culture room through the bottom wall portion using the culture device according to any one of Claims 1 to 10 set on an inverted microscope.

12. A culture chip comprising:

a membrane member having liquid permeability or liquid-containing substance permeability; and a frame portion fixed to one surface of the membrane member.

13. The culture chip according to Claim 12, wherein the membrane member has light transmittance.

FIG. 1

FIG. 2

# FIG. 3

# FIG. 4

## FIG. 5

FIRST DAY OF CULTURE
500 μm

CULTURED FOR 1 WEEK
500 μm

CULTURED FOR 2 WEEKS
500 μm

CULTURED FOR 3 WEEKS
500 μm

CULTURED FOR 4 WEEKS
500 μm

FIG. 6

## FIG. 7

## FIG. 8

| | PC PORE DIAMETER (μm) | |
|---|---|---|
| | 0.4 | 10 |
| BRIGHT FIELD | C | A |
| RFP | A | A |

FIG. 9

PC PORE DIAMETER 0.4 $\mu$m

BRIGHT FIELD

200 µm

RFP (Mitochondria)

200 µm

## FIG. 10

PC PORE DIAMETER 10 μm

BRIGHT FIELD

RFP (Mitochondria)

## FIG. 11

|  | PET PORE DIAMETER (μm) | |
|---|---|---|
|  | 0.45 | 3 |
| BRIGHT FIELD | S | B |
| RFP | A | A |

FIG. 12

PET PORE DIAMETER 0.45 μm

BRIGHT FIELD

RFP (Mitochondria)

200 μm

200 μm

FIG. 13

PET PORE DIAMETER 3 μm

BRIGHT FIELD

200 μm

RFP (Mitochondria)

200 μm

FIG. 14

| MATERIAL | PORE DIAMETER (μm) | POROSITY (%) | STATE OF MEMBRANE (LIGHT TRANSMITTANCE) |
|---|---|---|---|
| PC | 0. 40 | 18. 8 | MILKY WHITE |
| PC | 10. 0 | 5. 0~20. 0 | COLORLESS AND TRANSPARENT |
| PET | 0. 45 | 0. 6 | COLORLESS AND TRANSPARENT |
| PET | 3. 0 | 5. 7 | SLIGHTLY TRANSPARENT |

FIG. 15

## FIG. 16

COMPARATIVE EXAMPLE — PDMS

## FIG. 17

TPX — Lumox

# FIG. 18

# FIG. 19

# FIG. 20

FIG. 21

## INTERNATIONAL SEARCH REPORT

| | International application No. |
|---|---|
| | **PCT/JP2023/039902** |

**A. CLASSIFICATION OF SUBJECT MATTER**

*C12M 1/00*(2006.01)i; *C12M 1/34*(2006.01)i; *C12M 3/00*(2006.01)i; *C12N 5/076*(2010.01)i
FI:   C12M1/00 A; C12M1/34 A; C12M3/00 A; C12N5/076

According to International Patent Classification (IPC) or to both national classification and IPC

**B. FIELDS SEARCHED**

Minimum documentation searched (classification system followed by classification symbols)

C12M1/00; C12M1/34; C12M3/00; C12N5/076

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | 小川毅彦, 器官培養1. In vitro精子形成を用いた精巣毒性試験法の可能性, 谷本学校毒性質問箱, 26 September 2020, vol. 2020, no. 22, pp. 1-5, (OGAWA, Takehiko. Bulletin of Safety Evaluation Forum.), non-official translation (Organ culture 1. Possibility of testicular toxicity test method using in vitro spermatogenesis)<br>      pages 3-5, columns 5-8 | 1-13 |
| Y | KOMEYA, M. et al. In vitro spermatogenesis in two-dimensionally spread mouse testis tissues. Reproductive medicine and biology. 13 August 2019, vol. 18, no. 4, pp. 362-369<br>      abstract, columns 2.1, 2.3, 3.1-3.4 | 1-13 |
| Y | JP 2015-231354 A (FOUND PROMOTION IND SCIENCE) 24 December 2015 (2015-12-24)<br>      abstract, claims, paragraphs [0001], [0007]-[0010], [0025]-[0035], [0076]-[0091], fig. 1-13<br>      (Family: none) | 1-13 |
| Y | KOMEYA, M. et al. Long-term ex vivo maintenance of testis tissues producing fertile sperm in a microfluidic device. Scientific reports. 19 February 2016, vol. 6, article no. 21472 (pp. 1-10)<br>      abstract, results, fig. 1-4 | 1-13 |

☑ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2023/039902**

**C.    DOCUMENTS CONSIDERED TO BE RELEVANT**

| Category* | Citation of document, with indication, where appropriate, of the relevant passages | Relevant to claim No. |
|---|---|---|
| X | JP 2011-125328 A (NIKON CORP) 30 June 2011 (2011-06-30)<br>abstract, claims, paragraphs [0001], [0007]-[0010], [0012]-[0034], [0037], [0059]-[0063], fig. 1-14 | 1, 2, 4-6, 9-13 |
| A | <br>(Family: none) | 3, 7, 8 |
| A | JP 2008-109883 A (OSAKA UNIV) 15 May 2008 (2008-05-15)<br>abstract, claims, fig. 1, 2, 6<br>(Family: none) | 1-13 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022177449 A
- JP 2022025134 A
- JP 2013169685 A
- WO 2006054613 A
- WO 2014050817 A